(19) Europäisches Patentamt
European Patent Office
Office européen des brevets

(11) **EP 4 697 208 A1**

(12) **EUROPEAN PATENT APPLICATION**
published in accordance with Art. 153(4) EPC

(43) Date of publication:
**18.02.2026 Bulletin 2026/08**

(21) Application number: **24869773.2**

(22) Date of filing: **24.05.2024**

(51) International Patent Classification (IPC):
**G06F 18/10** (2023.01)

(86) International application number:
**PCT/CN2024/095337**

(87) International publication number:
**WO 2025/066208 (03.04.2025 Gazette 2025/14)**

(84) Designated Contracting States:
**AL AT BE BG CH CY CZ DE DK EE ES FI FR GB GR HR HU IE IS IT LI LT LU LV MC ME MK MT NL NO PL PT RO RS SE SI SK SM TR**
Designated Extension States:
**BA**
Designated Validation States:
**GE KH MA MD TN**

(30) Priority: **27.09.2023 CN 202311265053**

(71) Applicants:
• **Kingfar International Inc.**
**Beijing 100085 (CN)**

• **Nanjing Kingfar Health Technology Inc.**
**Nanjing, Jiangsu 211112 (CN)**

(72) Inventors:
• **ZHAO, Qichao**
**Beijing 100085 (CN)**
• **YANG, Ran**
**Beijing 100085 (CN)**
• **WANG, Qingju**
**Beijing 100085 (CN)**

(74) Representative: **DREISS Patentanwälte PartG mbB**
**Friedrichstraße 6**
**70174 Stuttgart (DE)**

(54) **HRV-DATA PREPROCESSING METHOD AND APPARATUS, AND ELECTRONIC DEVICE**

(57) The present disclosure relates to an HRV-data preprocessing method and apparatus, and an electronic device. The method includes: obtaining HRV data corresponding to a sliding window; determining a variance of a plurality of target peaks corresponding to the HRV data, and determining HRV data having the variance that falls within a predetermined variance range as first target HRV data; determining an RR interval sequence of the first target HRV data based on the plurality of target peaks, determining whether each RR interval in the RR interval sequence falls within a predetermined heartbeat time interval range, and determining second target HRV data; and extracting a time-domain feature, a frequency-domain feature, and a nonlinear feature of the second target HRV data. By filtering out abnormal data twice, the second target HRV data with high accuracy and in line with a human heartbeat characteristic is obtained, enabling an extraction of precise HRV features.

FIG. 1

**Description**

**CROSS-REFERENCE TO RELATED APPLICATION**

This application claims priority to Chinese Patent Application No. 202311265053.1, titled "HRV-DATA PREPROCESSING METHOD AND APPARATUS, AND ELECTRONIC DEVICE" and filed on September 27, 2023 with the China National Intellectual Property Administration, the entire contents of which are incorporated herein by reference.

**FIELD**

The present disclosure relates to the field of machine learning technologies, and more particularly, to an HRV-data preprocessing method and apparatus, and an electronic device.

**BACKGROUND**

Biological signal classification is an important diagnostic tool in biomedical applications, which can help medical experts automatically classify monitored biological signal samples into normal biological signals and abnormal biological signals. Most biological signals are inherently random and non-stationary, meaning their values are time-dependent and their statistics change at different time points.

Heart Rate Variability (HRV) is a type of important biological signal data. HRV refers to variations in time between heartbeats. Clinical practice has proven that HRV can serve as an independent predictor of a risk of sudden cardiac death, holding significant importance in evaluating the prognosis of cardiovascular diseases and predicting acute cardiovascular events. Generally, after preprocessing a large amount of collected raw physiological data, HRV feature values can be extracted. Based on the extracted feature values, machine learning can be applied to train models for cognitive load, etc., which can be used to predict states such as fatigue and disease.

However, conventional HRV signal data sources are electrocardiogram data or data from electrocardiographic monitoring equipment. Due to issues such as insufficient accuracy of a collection device or significant short-term changes in emotions or movements of individuals being monitored, some abnormal data exist in the collected HRV signal data. In the related art, a feature extraction is directly performed on the obtained raw HRV data, which results in abnormal data or interference data in extracted HRV data features, affecting accuracy of disease prediction based on HRV data features in clinical settings.

**SUMMARY**

To solve a problem in the related art of low accuracy in HRV feature data, the present disclosure provides an HRV-data preprocessing method and apparatus, and an electronic device.

In a first aspect, the present disclosure provides an HRV-data preprocessing method, which adopts the following technical solutions. The HRV-data preprocessing method includes: obtaining HRV data corresponding to a sliding window; determining, based on the HRV data, a plurality of target peaks corresponding to the HRV data, calculating a variance of the plurality of target peaks, and determining HRV data having the variance that falls within a predetermined variance range as first target HRV data; determining an RR interval sequence of the first target HRV data based on the plurality of target peaks of the first target HRV data, determining whether each RR interval in the RR interval sequence falls within a predetermined heartbeat time interval range, and determining, from the first target HRV data, HRV data for which all RR intervals fall within the predetermined heartbeat time interval range as second target HRV data; and extracting a time-domain feature, a frequency-domain feature, and a nonlinear feature of the second target HRV data.

By adopting the above technical solutions, the HRV data corresponding to the sliding window is obtained. The plurality of target peaks corresponding to the HRV data are determined. The variance of the plurality of target peaks is calculated. Filtering is performed on the HRV data based on the variance corresponding to the HRV data, which can exclude HRV data having a large variance, i.e., data exhibiting significant fluctuations that may be abnormal, obtaining the first target HRV data having high accuracy and relative stability that falls within the predetermined variance range. The RR interval sequence of the first target HRV data is determined based on the plurality of target peaks of the first target HRV data. In the RR interval sequence, the RR interval represents a time difference between two adjacent peaks, indicating a time interval between two consecutive heartbeats in a human body. By applying the predetermined heartbeat time interval range, the first target HRV data corresponding to abnormal heartbeats can be excluded, obtaining normal data, i.e., the second target HRV data. A feature extraction is performed on each piece of the second target HRV data. Through the variance and the predetermined heartbeat time interval range, two rounds of abnormal data filtering are conducted, leading to the second target HRV data having high accuracy that conforms to human heartbeat characteristics. By performing the feature extraction based on the second target HRV data, precise HRV features can be obtained.

In an optional example, the present disclosure can be further configured as followings. The determining, based on the HRV data, the plurality of target peaks corresponding to the HRV data includes: determining a plurality of initial peaks in the HRV data based on a minimum cardiac cycle point count, the minimum cardiac cycle point count representing a quantity of data points included in one heartbeat in the HRV data; determining a first upper envelope and a first lower envelope of the HRV data; determining a voltage threshold of the HRV data based on a percentage threshold, the first upper envelope, and the first lower envelope; and selecting, from the plurality of initial peaks, peaks exceeding the voltage threshold to obtain the plurality of target peaks of the HRV data.

By adopting the above technical solutions, a peak represents a maximum voltage amplitude of one heartbeat. The peaks corresponding to normal HRV data fall within a predetermined range. The plurality of initial peaks in the HRV data are determined based on the minimum cardiac cycle point count. The voltage threshold of the HRV data is calculated based on the percentage threshold and the first upper envelope and the first lower envelope of the HRV data. The voltage threshold represents a minimum peak that conforms to human physiological characteristics. By using the voltage threshold, peaks that conform to the human physiological characteristics can be selected from the plurality of initial peaks as the target peaks, making the obtained target peaks more accurate.

In an optional example, the present disclosure can be further configured as followings. The obtaining the HRV data corresponding to the sliding window includes: obtaining first initial HRV data corresponding to the sliding window, and determining a second upper envelope and a second lower envelope of the first initial HRV data; determining a maximum amplitude difference between the second upper envelope and the second lower envelope; determining an amplitude adjustment ratio of each data point in the first initial HRV data based on a second upper envelope value and a second lower envelope value that correspond to each data point in the first initial HRV data, and the maximum amplitude difference; and performing an amplitude adjustment on each data point in the first initial HRV data based on the amplitude adjustment ratio corresponding to the data point to obtain the HRV data.

By adopting the above technical solutions, the first initial HRV data corresponding to the sliding window is obtained. The second upper envelope and the second lower envelope of the first initial HRV data and the maximum amplitude difference between the second upper envelope and the second lower envelope are determined. The amplitude adjustment ratio of each data point in the overall initial HRV data is determined based on the second upper envelope value and the second lower envelope value that correspond to each data point in the first initial HRV data, and the maximum amplitude difference. Each data point is adjusted based on the amplitude adjustment ratio, in such a manner that an amplitude of the adjusted first initial HRV data remains within the original second upper and lower envelopes. An amplitude of a data point having an excessively high amplitude is reduced, while an amplitude of a data point having an excessively low amplitude is increased. Thus, the amplitudes of all data points in the first initial HRV data are adjusted into a reasonable amplitude range, leading to smoothed and valid HRV data.

In an optional example, the present disclosure can be further configured as followings. The performing the amplitude adjustment on each data point in the first initial HRV data based on the amplitude adjustment ratio corresponding to the data point to obtain the HRV data includes: performing the amplitude adjustment on each data point in the first initial HRV data based on the amplitude adjustment ratio corresponding to the data point to obtain amplitude-adjusted first HRV data; performing an anomaly detection on the amplitude-adjusted first HRV data to determine an anomalous point; and correcting a value of the anomalous point based on a mean or a median corresponding to a plurality of data points preceding or following the anomalous point to obtain the HRV data.

By adopting the above technical solutions, the anomaly detection is performed on the amplitude-adjusted first HRV data to determine the anomalous point. The value of the anomalous point is corrected based on a mean or a median of data surrounding the anomalous point. In this way, an anomalous value of the anomalous point can return to a normal value, ensuring accuracy and smoothness of the HRV data.

In an optional example, the present disclosure can be further configured as followings. The performing the anomaly detection on the amplitude-adjusted first HRV data to determine the anomalous point includes: determining, in the amplitude-adjusted first HRV data, a first difference between a target data point and a preceding data point and a second difference between the target data point and a following data point, the target data point being any data point in the first HRV data; calculating a first ratio of the first difference to the target data point and a second ratio of the second difference to the target data point; and when each of the first ratio and the second ratio for the target data point is greater than a predetermined ratio threshold, determining the target data point as the anomalous point.

By adopting the above technical solutions, the first ratio and the second ratio for the target data point in the amplitude-adjusted first HRV data are determined. When each of the first ratio and the second ratio for the target data point exceeds the predetermined ratio threshold, it is indicated that the target data point has undergone a sudden numerical change compared with surrounding data points, and thus the target data point is identified as the anomalous point. By analyzing a change of the target data point relative to the surrounding data points, the anomalous point can be accurately determined.

In an optional example, the present disclosure can be further configured as followings. The performing the anomaly detection on the amplitude-adjusted first HRV data to determine the anomalous point includes: determining a median of the amplitude-adjusted first HRV data, and determining a median deviation of a target data point in the amplitude-adjusted first HRV data based on the median; and when the median deviation of the target data point is greater than a predetermined deviation threshold, determining the target data point as the anomalous point.

By adopting the above technical solutions, the median of the amplitude-adjusted first HRV data is determined. The median deviation of the target data point in the amplitude-adjusted first HRV data is determined based on the median. When the median deviation of the target data point is greater than the predetermined deviation threshold, it is indicated that a degree to which the target data point deviates from the median of the first HRV data exceeds a normal range, and thus the target data point is identified as the anomalous point. In this way, the anomalous point with excessive deviation from the median of the data can be accurately screened out.

In an optional example, the present disclosure can be further configured as followings. The obtaining the first initial HRV data corresponding to the sliding window includes: obtaining second initial HRV data corresponding to the sliding window; performing a fast Fourier transform on a time-domain signal corresponding to the second initial HRV data to obtain a frequency-domain signal corresponding to the second initial HRV data; determining whether the frequency-domain signal corresponding to the second initial HRV data is a valid frequency-domain signal based on a predetermined frequency-domain range; and performing segmented filtering on the valid frequency-domain signal when the frequency-domain signal corresponding to the second initial HRV data is the valid frequency-domain signal, integrating the segmented-filtered valid frequency-domain signal, and performing an inverse fast Fourier transform on the integrated signal to obtain filtered first initial HRV data.

By adopting the above technical solutions, subsequent to a conversion of the time-domain signal into the frequency-domain signal, the valid frequency-domain signal is determined based on the predetermined frequency-domain range, which can exclude data having an abnormal frequency and retain valid data. Also, by performing the segmented filtering on the valid data for a noise reduction, noise interference can be accurately removed, improving accuracy of the first initial HRV data.

In an optional example, the present disclosure can be further configured as followings. The extracting the nonlinear feature of the second target HRV data includes: drawing a Poincaré scatter plot and a difference scatter plot based on the second target HRV data; determining a standard deviation of a major axis and a standard deviation of a minor axis of an ellipse fitted to the Poincaré scatter plot; and determining a quantity of points in each of a first quadrant and a third quadrant of the difference scatter plot. The nonlinear feature includes the standard deviation of the major axis of the ellipse, the standard deviation of the minor axis of the ellipse, the quantity of points in the first quadrant, and the quantity of points in the third quadrant.

By adopting the above technical solutions, the Poincaré scatter plot and the difference scatter plot are drawn based on the second target HRV data. The standard deviation of the major axis and the standard deviation of the minor axis of the ellipse fitted to the Poincaré scatter plot can reflect parasympathetic nervous activity of the human body. The quantity of points in the first quadrant and the quantity of points in the third quadrant that are obtained from the difference scatter plot can reflect changes in two consecutive cardiac intervals, indicating a heart rate and sympathetic nervous activity. Based on these nonlinear indicators, a physiological state of the human body can be assessed.

In an optional example, the present disclosure can be further configured as followings. The plurality of target peaks are peaks in the HRV data that exceed a voltage threshold; and the voltage threshold is determined by: determining a maximum amplitude difference between a first upper envelope and a first lower envelope of the HRV data, determining a product of the maximum amplitude difference and a predetermined percentage threshold, and determining a sum of the product and an amplitude of the first lower envelope corresponding to the maximum amplitude difference as the voltage threshold.

In a second aspect, the present disclosure provides an HRV-data preprocessing apparatus, which adopts the following technical solutions. The HRV-data preprocessing apparatus includes: an obtaining module configured to obtain HRV data corresponding to a sliding window; a first processing module configured to determine, based on the HRV data, a plurality of target peaks corresponding to the HRV data, calculate a variance of the plurality of target peaks, and determine HRV data having the variance that falls within a predetermined variance range as first target HRV data; a second processing module configured to determine an RR interval sequence of the first target HRV data based on the plurality of target peaks of the first target HRV data, determine whether each RR interval in the RR interval sequence falls within a predetermined heartbeat time interval range, and determine, from the first target HRV data, HRV data for which all RR intervals fall within the predetermined heartbeat time interval range as second target HRV data; and an extraction module configured to extract a time-domain feature, a frequency-domain feature, and a nonlinear feature of the second target HRV data.

In a third aspect, the present disclosure provides an electronic device, which adopts the following technical solutions. The electronic device includes: one or more processors; a memory; and one or more applications stored in the memory and configured to be executed by the one or more processors to implement the HRV-data preprocessing method according to any one of the embodiments in the first aspect.

In a fourth aspect, the present disclosure provides a computer-readable storage medium, which adopts the following technical solutions. The computer-readable storage medium stores a computer program or computer instructions. The computer program or the computer instructions, when executed by a processor, implement the HRV-data preprocessing method according to any one of the embodiments in the first aspect.

In a fifth aspect, the present disclosure provides an apparatus. The apparatus includes: a processor configured to invoke and execute a computer program from a memory, enabling a device equipped with the apparatus to perform the HRV-data preprocessing method according to any one of the embodiments in the first aspect.

In a sixth aspect, the present disclosure provides a computer program product. The computer program product includes a computer program or computer instructions. The computer program or the computer instructions, when executed by a processor, implement the HRV-data preprocessing method according to any one of the embodiments in the first aspect.

In summary, the present disclosure has the following advantageous technical effects.

In the present disclosure, the HRV data corresponding to the sliding window is obtained. The plurality of target peaks corresponding to the HRV data are determined. The variance of the plurality of target peaks is calculated. The filtering is performed on the HRV data based on the variance corresponding to the HRV data, which can exclude the HRV data having the large variance, i.e., the data exhibiting significant fluctuations that may be abnormal, obtaining the first target HRV data having high accuracy and relative stability that falls within the predetermined variance range. The RR interval sequence of the first target HRV data is determined based on the plurality of target peaks of the first target HRV data. In the RR Interval sequence, the RR interval represents the time difference between two adjacent peaks, indicating the time interval between two consecutive heartbeats in the human body. By applying the predetermined heartbeat time interval range, the first target HRV data corresponding to the abnormal heartbeats can be excluded, obtaining the normal data, i.e., the second target HRV data. The feature extraction is performed on each piece of the second target HRV data. Through the variance and the predetermined heartbeat time interval range, the two rounds of abnormal data filtering are conducted, leading to the second target HRV data having high accuracy that conforms to the human heartbeat characteristics. By performing the feature extraction based on the second target HRV data, the precise HRV features can be obtained.

## BRIEF DESCRIPTION OF THE DRAWINGS

FIG. 1 is a schematic flowchart of an HRV-data preprocessing method according to an embodiment of the present disclosure.

FIG. 2 is a partial time-domain signal waveform according to an embodiment of the present disclosure.

FIG. 3 is a schematic diagram of a first upper envelope and a first lower envelope according to an embodiment of the present disclosure.

FIG. 4 is a schematic diagram of a target peak according to an embodiment of the present disclosure.

FIG. 5 is a schematic diagram of a waveform before an amplitude adjustment according to an embodiment of the present disclosure.

FIG. 6 is a schematic diagram of a waveform after an amplitude adjustment according to an embodiment of the present disclosure.

FIG. 7 is a comparative diagram of waveforms before and after an amplitude adjustment according to an embodiment of the present disclosure.

FIG. 8 is a schematic diagram of a power spectrum waveform according to an embodiment of the present disclosure.

FIG. 9 is a Poincaré scatter plot according to an embodiment of the present disclosure.

FIG. 10 is a schematic structural diagram of an HRV-data preprocessing apparatus according to an embodiment of the present disclosure.

FIG. 11 is a schematic structural diagram of an electronic device according to an embodiment of the present disclosure.

## DETAILED DESCRIPTION

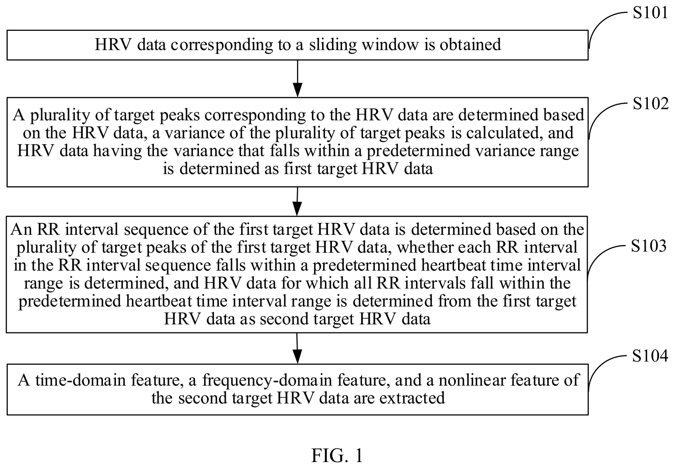

The present disclosure is described in further detail below in conjunction with FIG. 1 to FIG. 11.

Specific embodiments are merely an illustration of the present disclosure and is not intended to limit the present disclosure. After reading this specification, those skilled in the art can make modifications to these embodiments as needed without involving inventive contribution, but any such modifications that fall within the scope of the claims of the present disclosure are protected by patent law.

In order to make the objectives, technical solutions, and advantages of the embodiments of the present disclosure more apparent, technical solutions according to the embodiments of the present disclosure will be described clearly and completely below in combination with accompanying drawings of the embodiments of the present disclosure. Obviously, the embodiments described below are only a part of the embodiments of the present disclosure, rather than all embodiments of the present disclosure. On a basis of the embodiments of the present disclosure, all other embodiments obtained by those skilled in the art without creative labor shall fall within the protection scope of the present disclosure.

In addition, the term "and/or" in the present disclosure only represents an association relationship between correlated objects, including three relationships. For example, "A and/or B" may mean three situations: A only, B only, or both A and B. In addition, the character "/" in the present disclosure generally represents an "or" relationship between the correlated objects preceding and succeeding the symbol, unless otherwise specified.

For ease of understanding, an explanation of terms involved in the present disclosure is provided below.

RR interval: defined as a time difference between two consecutive peaks, also known as NN interval or Inter-Beat Interval (IBI). The RR interval is typically measured in milliseconds (ms) and serves as crucial data for calculating HRV indicators.

Heart rate: represents a quantity of heartbeats per minute in a normal person at rest.

ECG: stands for electrocardiogram, which records electrical activity changes produced by the heart during each cardiac cycle from a surface of a human body using an electrocardiograph.

PPG: stands for photoplethysmogram, which uses a photoelectric sensor to detect variations in reflected light intensity after absorption by human blood and tissues, recording changes in blood vessel volume during the cardiac cycle.

HRV data can be a time series of beat-to-beat intervals extracted from normal human ECG signals or pulse signals (PPG signals). A signal waveform of the HRV data is a time-domain signal, representing changes of a voltage generated by human heartbeats over time. In the time-domain signal waveform, the horizontal axis represents time, and the vertical axis represents voltage.

The embodiments of the present disclosure provide an HRV-data preprocessing method. As illustrated in FIG. 1, the method according to the embodiments of the present disclosure can be performed by an electronic device. The electronic device may be a server or a terminal device. The server may be an independent physical server, a server cluster formed by a plurality of physical servers or a distributed system, or a cloud server providing cloud computing services. The terminal device may be a smartphone, a tablet computer, a laptop, a desktop computer, etc., but is not limited thereto. The terminal device and the server may be directly or indirectly connected via a wired or wireless communication method, which is not limited in the embodiments of the present disclosure.

The HRV-data preprocessing method according to the embodiments of the present disclosure primarily addresses an issue related to accuracy of a feature extraction from raw physiological signals during a real-time prediction. Due to problems such as insufficient precision of a collection device or a possible deviation in wearing, the collected HRV data may contain abnormal data. According to the embodiments of the present disclosure, the HRV data is preprocessed to discard such abnormal data, improving accuracy of the HRV data to facilitate an extraction of more precise data features. The method includes operations at blocks.

At S101, HRV data corresponding to a sliding window is obtained.

In the embodiments of the present disclosure, real-time HRV data is obtained, and the real-time HRV data is segmented based on the sliding window to obtain the HRV data corresponding to the sliding window.

Specifically, as the sliding window slides by a specific sliding duration, data of the sliding window duration is segmented from the obtained real-time HRV data. When a duration of the obtained real-time HRV data exceeds the sliding window duration, the real-time HRV data can be segmented using the sliding window. It is assumed that, the sliding window duration is set to 15 seconds, and the specific sliding duration of the sliding window is 1 second. When the duration of the obtained real-time HRV data exceeds 15 seconds, the sliding window can perform a 1-st segmentation on the real-time HRV data to obtain HRV data corresponding to 0 second to 15-th second of the sliding window. When the duration of the real-time HRV data exceeds a sum of the sliding window duration and the specific sliding duration, i.e., 16 seconds, a 2-nd segmentation is performed on the real-time HRV data using the sliding window to obtain HRV data corresponding to 1-st second to 16-th second of the sliding window. By analogy, each time the sliding window slides, 15 seconds of HRV data can be obtained, ultimately resulting in a plurality of segments of HRV data.

The specific sliding duration and the sliding window duration are not limited in the embodiments of the present disclosure, and can be set by a user as desired.

The real-time HRV data is segmented based on the specific sliding duration using the sliding window to obtain the HRV data corresponding to the sliding window. By segmenting the data with the sliding window, a sequential relationship within a time series can be preserved, allowing a long sequence to be divided into a plurality of short window data, i.e., the HRV data. Adjacent HRV data exhibit data redundancy. Thus, even if some window data is removed during a subsequent data processing, relative integrity of the overall data can still be guaranteed. Through the window data, fine changes and fluctuations can be captured. Consequently, a data removal can be performed based on abnormalities in these fine changes and fluctuations, making the finally retained data more precise.

At S102, a plurality of target peaks corresponding to the HRV data are determined based on the HRV data, a variance of the plurality of target peaks is calculated, and HRV data having the variance that falls within a predetermined variance range is determined as first target HRV data.

In the embodiments of the present disclosure, it is assumed that n target peaks correspond to a certain sliding window, denoted as R1, R2, ..., Rn. A mean avg of the n target peaks is calculated. An expression of the mean avg is:

$$avg = \frac{R1 + R2 + \cdots + Rn}{n}.$$

A variance $s^2$ of the n target peaks is calculated. An expression of the variance $s^2$ is:

$$s^2 = \frac{\sum_{i=1}^{n} \left( \frac{R_i}{avg} - 1 \right)^2}{n}.$$

In the expression, n represents a quantity of target peaks, $R_i$ represents an i-th target peak among the n target peaks, and avg represents the mean of the n target peaks.

Whether the variance falls within the predetermined variance range is determined. When the variance does not fall within the predetermined variance range, it is indicated that a network delay of the collection device worn by the user or shaking of the worn collection device may have caused significant fluctuations in the target peaks corresponding to the HRV data. To ensure the accuracy of the overall data, the HRV data is discarded. Only the HRV data having the variance that falls within the predetermined variance range is determined as the first target HRV data. The predetermined variance range represents a degree of fluctuation of the target peaks and can be set to range from 0 to 1 as desired.

At S103, an RR interval sequence of the first target HRV data is determined based on the plurality of target peaks of the first target HRV data, whether each RR interval in the RR interval sequence falls within a predetermined heartbeat time interval range is determined, and HRV data for which all RR intervals fall within the predetermined heartbeat time interval range is determined from the first target HRV data as second target HRV data.

In the embodiments of the present disclosure, for the first target HRV data, respective time points corresponding to the plurality of target peaks are obtained. A time difference between two adjacent target peaks in the first target HRV data is calculated to obtain the RR interval sequence corresponding to the first target HRV data. It is assumed that, the quantity of target peaks in the first target HRV data is n+1, and horizontal coordinate time points corresponding to the target peaks are R1, R2, ..., Rn+1, respectively. By calculating the time difference between every two adjacent target peaks, a quantity of RR intervals obtained is n, and the corresponding RR interval sequence can be expressed as [RR1, RR2, ..., RRn], where RRn = Rn+1-Rn.

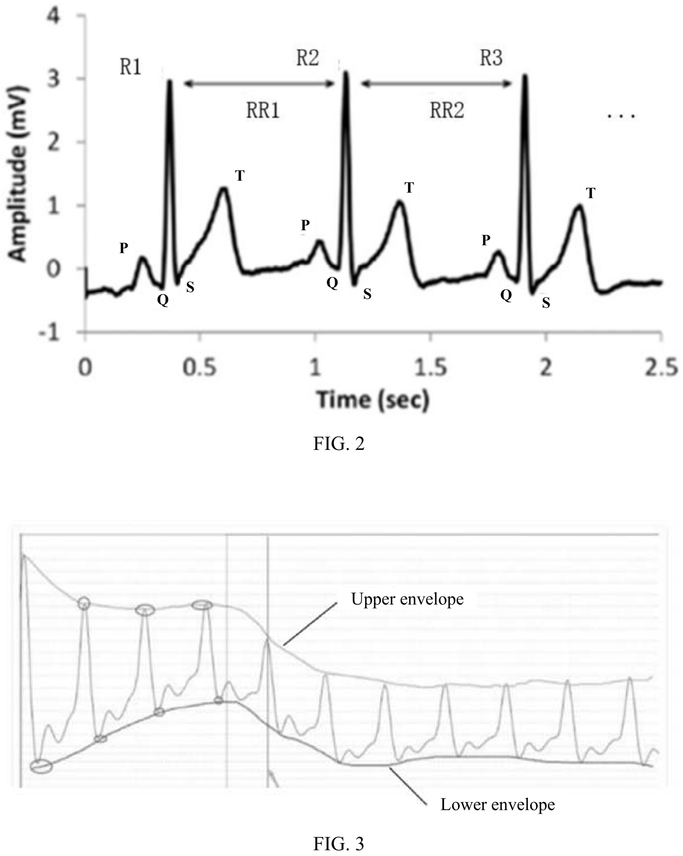

FIG. 2 illustrates a partial time-domain signal waveform corresponding to the first target HRV data, where the horizontal axis represents time and the vertical axis represents a voltage amplitude corresponding to time. Peaks indicate a maximum voltage generated by one heartbeat. FIG. 2 illustrates three target peaks, whose corresponding horizontal coordinates are R1, R2, and R3 respectively. The time difference between R1 and R2 is R2-R1. The RR interval formed by R1 and R2 is RR1. Similarly, the RR interval formed by R2 and R3 is: RR2=R3-R2. In FIG. 2, the QST complex represents ventricular depolarization, the P wave represents atrial excitation, the T wave represents ventricular repolarization, the QT interval represents time from ventricular depolarization to complete repolarization, and the ST segment represents a period after a completion of ventricular depolarization but before a start of repolarization.

The predetermined heartbeat time interval range represents a maximum value of the RR interval under normal conditions, which can be determined based on the age of a subject. If an RR interval that does not fall within the predetermined heartbeat time interval exists in the RR interval sequence corresponding to the first target HRV data, it is indicated that abnormal data has occurred in the first target HRV data. The first target HRV data corresponding to the abnormal data is discarded. The first target HRV data for which all RR intervals fall within the predetermined heartbeat time interval range is determined as the second target HRV data.

At S104, a time-domain feature, a frequency-domain feature, and a nonlinear feature of the second target HRV data are extracted.

Thus, in the embodiments of the present disclosure, the HRV data corresponding to the sliding window is obtained. The plurality of target peaks corresponding to the HRV data are determined. The variance of the plurality of target peaks is calculated. Filtering is performed on the HRV data based on the variance corresponding to the HRV data, which can exclude HRV data having a large variance, i.e., data exhibiting significant fluctuations that may be abnormal, obtaining the first target HRV data having high accuracy and relative stability that falls within the predetermined variance range. The RR interval sequence of the first target HRV data is determined based on the plurality of target peaks of the first target HRV data. In the RR interval sequence, the RR interval represents a time difference between two adjacent peaks, indicating a time interval between two consecutive heartbeats in a human body. By applying the predetermined heartbeat time interval range, the first target HRV data corresponding to abnormal heartbeats can be excluded, obtaining normal data, i.e., the second target HRV data. A feature extraction is performed on each piece of the second target HRV data. Through the variance and the predetermined heartbeat time interval range, two rounds of abnormal data filtering are conducted, leading to the second target HRV data having high accuracy that conforms to human heartbeat characteristics. By performing the feature extraction based on the second target HRV data, precise HRV features can be obtained.

In a possible implementation of the embodiments of the present disclosure, determining, based on the HRV data, the plurality of target peaks corresponding to the HRV data includes: determining a plurality of initial peaks in the HRV data based on a minimum cardiac cycle point count, in which the minimum cardiac cycle point count represents a quantity of data points included in one heartbeat in the HRV data; determining a first upper envelope and a first lower envelope of the HRV data; determining a voltage threshold of the HRV data based on a percentage threshold, the first upper envelope, and the first lower envelope; and selecting, from the plurality of initial peaks, peaks exceeding the voltage threshold to obtain the plurality of target peaks of the HRV data.

In this embodiment, the minimum cardiac cycle point count represents a minimum quantity of data points corresponding to one heartbeat, which can be calculated through the following expression:

$$winSize = \frac{60}{maxRatePerMin1} \times freq.$$

In the expression, winSize represents the minimum cardiac cycle point count, and maxRatePerMin1 represents a maximum heart rate, i.e., a maximum quantity of heartbeats per minute for a normal person at rest, which can be set as 120 based on human physiological characteristics; freq represents a sampling rate set when collecting original HRV data, indicating a quantity of data points collected per second; and 60 represents that one minute equals 60 seconds, which is used for a unit conversion.

In the embodiments of the present disclosure, all maximum points and all minimum points in the HRV data are determined. An HRV time-domain signal waveform (i.e., the HRV data) is segmented based on the minimum cardiac cycle point count, resulting in a plurality of data segments. A quantity of data points contained in each data segment is equal to the minimum cardiac cycle point count. For each data segment: when the data segment contains no maximum point, no initial peak is selected; when the data segment contains one maximum point, the one maximum point is determined as the initial peak; and when the data segment contains two or more maximum points, the one having a largest amplitude among these two or more maximum points is selected as the initial peak. Thus, the plurality of initial peaks corresponding to the HRV data are obtained.

Filtering is performed on the initial peaks using the voltage threshold to obtain target peaks that conform to the human physiological characteristics. Specifically, as illustrated in FIG. 3, all maximum points are connected by a smooth curve to form the first upper envelope of the HRV data. Between two adjacent maximum points, a smallest minimum point is selected as a trough. All troughs are connected by a smooth curve to form the first lower envelope of the HRV data.

An amplitude difference between the first upper envelope and the first lower envelope that correspond to each data point in the HRV data is determined. An expression of the difference is:

$$maxThresholdi = upperEnvelopei - underEnvelopei.$$

In the expression, maxThresholdi represents a difference between the first upper envelope and the first lower envelope that correspond to an i-th data point in the HRV data, upperEnvelopei represents an amplitude of the first upper envelope corresponding to the i-th data point in the HRV data, and underEnvelopei represents an amplitude of the first lower envelope corresponding to the i-th data point in the HRV data.

A maximum value maxThreshold1 of the amplitude difference between the first upper envelope and the first lower envelope is determined.

Alternatively, the plurality of target peaks are peaks in the HRV data that exceed a voltage threshold. The voltage threshold is determined by: determining a maximum amplitude difference between a first upper envelope and a first lower envelope of the HRV data, determining a product of the maximum amplitude difference and a predetermined percentage threshold, and determining a sum of the product and an amplitude of the first lower envelope corresponding to the maximum amplitude difference as the voltage threshold.

Specifically, the voltage threshold is calculated. The voltage threshold is also known as a percentage line value, which represents a minimum peak in the HRV data collected from a normal human body. An expression of the percentage line value Y is:

$$Y = maxThreshold1 \times thersholdPercent + underEnvelope.$$

In the expression, thresholdPercent represents the predetermined percentage threshold, which can be set based on historical HRV data, e.g., set to 70%; maxThreshold1 represents a maximum value of the amplitude difference between the first upper envelope and the first lower envelope; and underEnvelope represents an amplitude of the first lower envelope corresponding to maxThreshold1.

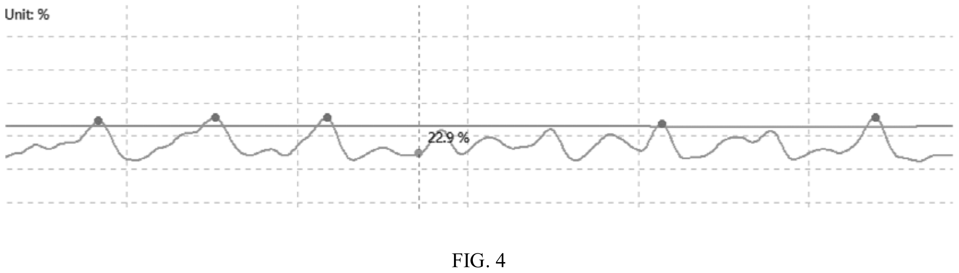

As illustrated in FIG. 4, the curve represents a time-domain signal waveform of the HRV data, and the straight line represents the percentage line, whose corresponding amplitude is the voltage threshold. In FIG. 4, initial peaks exceeding the voltage threshold are determined as target peaks.

Thus, in the embodiments of the present disclosure, a peak represents a maximum voltage amplitude of one heartbeat. The peaks corresponding to normal HRV data fall within a predetermined range. The plurality of initial peaks in the HRV data are determined based on the minimum cardiac cycle point count. The voltage threshold of the HRV data is calculated based on the percentage threshold and the first upper envelope and the first lower envelope of the HRV data. The voltage threshold represents a minimum peak that conforms to human physiological characteristics. By using the voltage threshold, peaks that conform to the human physiological characteristics can be selected from the plurality of initial peaks as the target peaks, making the obtained target peaks more accurate.

Further, to improve the accuracy of the HRV data, calculating the variance of the plurality of target peaks includes: calculating the time difference between two adjacent target peaks as the RR interval; determining whether the RR interval falls within a predetermined time interval range; when any RR interval falls outside a heartbeat time interval range, determining the HRV data as abnormal data; and when all RR intervals fall within the heartbeat time interval range, calculating the variance of the plurality of target peaks, and determining the HRV data having the variance that falls within the predetermined variance range as the first target data. The heartbeat time interval range refers to a heartbeat time interval that RR intervals corresponding to the HRV data collected from the normal human body should satisfy, and can be set to range from 0 second to 3 seconds.

In a possible implementation of the embodiments of the present disclosure, obtaining the HRV data corresponding to the sliding window includes: obtaining first initial HRV data corresponding to the sliding window, and determining a second upper envelope and a second lower envelope of the first initial HRV data; determining a maximum amplitude difference between the second upper envelope and the second lower envelope; determining an amplitude adjustment ratio of each data point in the first initial HRV data based on a second upper envelope value and a second lower envelope value that correspond to each data point in the first initial HRV data, and the maximum amplitude difference; and performing an amplitude adjustment on each data point in the first initial HRV data based on the amplitude adjustment ratio corresponding to the data point to obtain the HRV data.

Specifically, the second upper envelope and the second lower envelope of the first initial HRV data are determined. An envelope generation process can refer to the above process for generating the first upper envelope and the first lower envelope.

An expression of the amplitude adjustment ratio ratio of a current data point is:

$$\text{ratio} = \frac{h1 - h2}{\text{maxThreshold2}}.$$

In the expression, h1 represents an amplitude at the second upper envelope corresponding to the current data point, h2 represents an amplitude at the second lower envelope corresponding to the current data point, and maxThreshold2 represents a maximum value of the amplitude difference between the second upper envelope and the second lower envelope.

An expression of an adjusted amplitude h3 of the current data point is:

$$h3 = \frac{h - h2}{\text{ratio}}.$$

In the expression, h represents an amplitude of the current data point before an adjustment, h2 represents the amplitude at the second lower envelope corresponding to the current data point, and **ratio** represents the amplitude adjustment ratio of the current data point.

An amplitude of the original HRV data collected by the device is a voltage. The amplitude of the original HRV data can be converted from voltage to percentage, facilitating a processing and an analysis of the HRV data. Specifically, a ratio of a voltage amplitude of all HRV data points to a set value is determined as an updated amplitude of the data point, resulting in the first initial HRV data with the amplitude expressed in percentage. The set value is determined as desired, aiming to adjust, through the set value, the original voltage amplitudes within an appropriate percentage range.

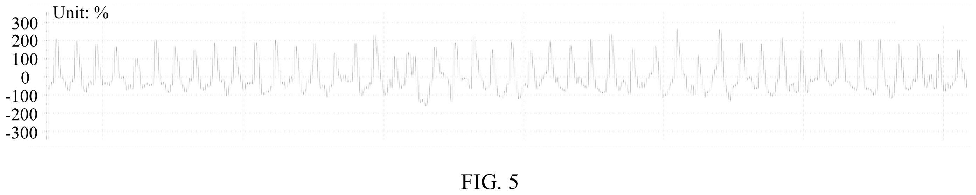

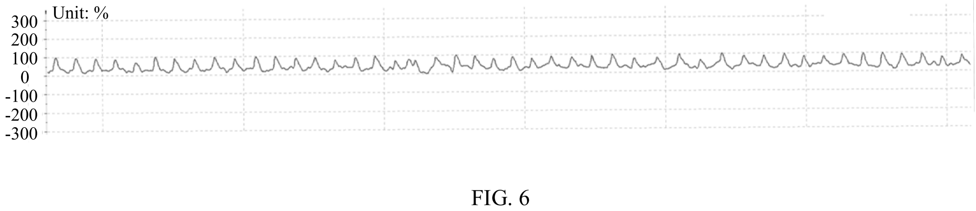

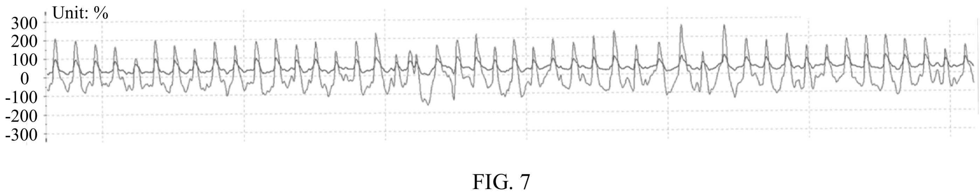

FIG. 5 is a waveform of the first initial HRV data before an amplitude adjustment, where the horizontal axis of the waveform represents time and the vertical axis represents amplitude in percentage. FIG. 6 is a waveform of the HRV data after an amplitude adjustment. FIG. 7 is a comparative diagram of waveforms before and after an amplitude adjustment. These figures reveal that, the waveform after the amplitude adjustment lies within a smaller amplitude range and is smoother.

Thus, in the embodiments of the present disclosure, the first initial HRV data corresponding to the sliding window is obtained. The second upper envelope and the second lower envelope of the first initial HRV data and the maximum amplitude difference between the second upper envelope and the second lower envelope are determined. The amplitude adjustment ratio of each data point in the overall initial HRV data is determined based on the second upper envelope value and the second lower envelope value that correspond to each data point in the first initial HRV data, and the maximum amplitude difference. Each data point is adjusted based on the amplitude adjustment ratio, in such a manner that an amplitude of the adjusted first initial HRV data remains within the original second upper and lower envelopes. An amplitude of a data point having an excessively high amplitude is reduced, while an amplitude of a data point having an excessively low amplitude is increased. Thus, the amplitudes of all data points in the first initial HRV data are adjusted into a reasonable amplitude range, leading to smoothed and valid HRV data.

In a possible implementation of the embodiments of the present disclosure, performing the amplitude adjustment on each data point in the first initial HRV data based on the amplitude adjustment ratio corresponding to the data point to obtain the HRV data includes: performing the amplitude adjustment on each data point in the first initial HRV data based on the amplitude adjustment ratio corresponding to the data point to obtain amplitude-adjusted first HRV data; performing an anomaly detection on the amplitude-adjusted first HRV data to determine an anomalous point; and correcting a value of the anomalous point based on a mean or a median corresponding to a plurality of data points preceding or following the anomalous point to obtain the HRV data.

The first initial HRV data is generally non-smooth. Data accuracy can be improved through detecting and correcting abnormal data. For the detected anomalous point, this embodiment provides two correction methods: mean correction and median correction.

Specifically, for each anomalous point, the mean or the median corresponding to the plurality of data points preceding or following the anomalous point is determined as a replacement value for the anomalous point. The plurality of data points may be a specific quantity of data points preceding or following the anomalous point, or may be data points corresponding to a specific duration preceding or following the anomalous point. The specific quantity and the specific duration are set as desired. Alternatively, the specific quantity is set to 75, and the specific duration is set to 2.5 seconds.

Thus, in the embodiments of the present disclosure, the anomaly detection is performed on the amplitude-adjusted first HRV data to determine the anomalous point. The value of the anomalous point is corrected based on a mean or a median of data surrounding the anomalous point. In this way, an anomalous value of the anomalous point can return to a normal value, ensuring accuracy and smoothness of the HRV data.

In a possible implementation of the embodiments of the present disclosure, performing the anomaly detection on the amplitude-adjusted first HRV data to determine the anomalous point includes: determining, in the amplitude-adjusted first HRV data, a first difference between a target data point and a preceding data point and a second difference between the target data point and a following data point, the target data point being any data point in the first HRV data; calculating a first ratio of the first difference to the target data point and a second ratio of the second difference to the target data point; and when each of the first ratio and the second ratio for the target data point is greater than a predetermined ratio threshold, determining the target data point as the anomalous point.

In the embodiments of the present disclosure, a percentage detection is used to detect anomalous points that exhibit sudden amplitude changes compared with adjacent data points. The predetermined ratio threshold represents a degree of amplitude change of the target data point relative to a preceding data point or a following data point, and can be set to 20% as desired.

Specifically, it is assumed that, an amplitude of the target data point is B, an amplitude of the preceding data point relative to the target data point is A, and an amplitude of the following data point relative to the target data point is C. The difference between the target data point and the preceding data point is the first difference B-A. The difference between the target data point and the following data point is the second difference B-C. An absolute value of a ratio of the first difference B-A to the target data point is the first ratio $|(B-A)/B \times 100\%1$. An absolute value of a ratio of the second difference B-C to the target data point is the second ratio $|(B-C)/B \times 100\%|$. When each of the first ratio and the second ratio for the target data point is greater than the predetermined ratio threshold, the target data point is determined as the anomalous point.

Thus, in the embodiments of the present disclosure, the first ratio and the second ratio for the target data point in the amplitude-adjusted first HRV data are determined. When each of the first ratio and the second ratio for the target data point exceeds the predetermined ratio threshold, it is indicated that the target data point has undergone a sudden numerical change compared with surrounding data points, and thus the target data point is identified as the anomalous point. By analyzing a change of the target data point relative to the surrounding data points, the anomalous point can be accurately determined.

In a possible implementation of the embodiments of the present disclosure, performing the anomaly detection on the amplitude-adjusted first HRV data to determine the anomalous point includes: determining a median of the amplitude-adjusted first HRV data, and determining a median deviation of a target data point in the amplitude-adjusted first HRV data based on the median; and when the median deviation of the target data point is greater than a predetermined deviation threshold, determining the target data point as the anomalous point.

In the embodiments of the present disclosure, the median of the amplitude-adjusted first HRV data is determined; an absolute value of a difference between each data point and the median is calculated to generate a set of differences; and a median medianDiff of the set of differences is determined.

The median deviation f of the target data point is calculated. An expression of the median deviation f is:

$$f = \frac{\text{amplitude of the target data point } - \text{median}}{k \times \text{medianDiff}}$$

In the expression, k represents a coefficient set empirically and has a value of 1.483.

When the median deviation of the target data point is greater than the predetermined deviation threshold, the target data point is determined as the anomalous point. The predetermined threshold represents a maximum degree to which the target data point deviates from the median of the data and may be set empirically to 4.

Thus, in the embodiments of the present disclosure, the median of the amplitude-adjusted first HRV data is determined. The median deviation of the target data point in the amplitude-adjusted first HRV data is determined based on the median. When the median deviation of the target data point is greater than the predetermined deviation threshold, it is indicated that a degree to which the target data point deviates from the median of the first HRV data exceeds a normal range, and thus the target data point is identified as the anomalous point. In this way, the anomalous point with excessive deviation from the median of the data can be accurately screened out.

In a possible implementation of the embodiments of the present disclosure, obtaining the first initial HRV data corresponding to the sliding window includes: obtaining second initial HRV data corresponding to the sliding window; performing a fast Fourier transform on a time-domain signal corresponding to the second initial HRV data to obtain a frequency-domain signal corresponding to the second initial HRV data; determining whether the frequency-domain signal corresponding to the second initial HRV data is a valid frequency-domain signal based on a predetermined frequency-domain range; and performing segmented filtering on the valid frequency-domain signal when the frequency-domain signal corresponding to the second initial HRV data is the valid frequency-domain signal, integrating the segmented-filtered valid frequency-domain signal, and performing an inverse fast Fourier transform on the integrated signal to obtain filtered first initial HRV data.

A main principle of filtering involves converting the time-domain signal into the frequency-domain signal in segments, performing filtering on the frequency-domain signal, and then converting the frequency-domain signal back to the time-domain signal after the filtering. The fast Fourier transform is performed on the obtained second initial HRV data corresponding to the sliding window to obtain the frequency-domain signal.

The frequency-domain signal of the HRV data is mainly concentrated within the predetermined frequency-domain range. A ratio of signal energy within the predetermined frequency-domain range to total frequency-domain energy is calculated. When the ratio is greater than the predetermined ratio threshold, it is indicated that the HRV data corresponding to the frequency-domain signal is valid data. Valid HRV data is selected for filtering to obtain the accurate first initial HRV data. A predetermined frequency-domain range for PPG signals may range from 0.5 Hz to 4 Hz. A predetermined frequency-domain range for ECG signals may range from 0.25 Hz to 45 Hz. The predetermined ratio threshold may be set empirically to 90%.

The segmented filtering is performed on the valid frequency-domain signal. An advantage of the segmented filtering is an absence of phase deviation, but the data cannot be too long, and a window length in real-time filtering cannot be too long either. Therefore, in the embodiments of the present disclosure, a window of a length 4,096 is selected as a filtering window, meaning that each filtering window represents a segment of HRV data containing 4,096 data points.

Further, window filtering can employ high-pass filtering. The high-pass filtering is configured to allow high-frequency signals to pass normally, and block or attenuate low-frequency signals below a set critical frequency. In this way, data spikes can be addressed, and power-line interference from an environment can be removed. The data spikes refer to random and transient peaks, jitter, or outliers in the data caused by issues such as unsatisfactory data source quality, sampling deviations, device failure, or other external factors. Due to interference from other devices or a fact that shaking is likely to occur when the collection device is an ear-clip sensor, a power-line interference signal may be generated. The power-line interference signal typically has a frequency of 50 Hz or 60 Hz and mainly manifest as sinusoidal waves or a superposition of other signals with sinusoidal waves that may occur during a signal measurement.

Thus, in the embodiments of the present disclosure, subsequent to a conversion of the time-domain signal into the frequency-domain signal, the valid frequency-domain signal is determined based on the predetermined frequency-domain range, which can exclude data having an abnormal frequency and retain valid data. Also, by performing the segmented filtering on the valid data for a noise reduction, noise interference can be accurately removed, improving accuracy of the first initial HRV data.

In a possible implementation of the embodiments of the present disclosure, prior to performing filtering on the second initial HRV data, wavelet decomposition is performed on the second initial HRV data corresponding to the sliding window. A wavelet coefficient generated by the wavelet decomposition contains important information of the HRV data. A wavelet coefficient corresponding to valid data subsequent to the wavelet decomposition is relatively large, while a wavelet coefficient corresponding to noise is relatively small. The wavelet coefficient of noise is smaller than that of a valid signal. By selecting an appropriate predetermined threshold for screening, the wavelet coefficient greater than the predetermined threshold is considered as a valid signal, while the wavelet coefficient smaller than the predetermined threshold is considered as noise. The wavelet coefficient is adjusted based on the predetermined threshold. The adjusted wavelet coefficient for each layer is subjected to wavelet reconstruction to obtain the wavelet-denoised second initial HRV data.

A quantity of layers for the wavelet decomposition is set as desired and may be set to five layers. The predetermined threshold is set based on a noise level in historical data and may be set to 30 db.

Further, adjusting the wavelet coefficient based on the predetermined threshold can involve setting the wavelet coefficient smaller than the predetermined threshold to zero, leaving the wavelet coefficient greater than or equal to the predetermined threshold unchanged, or replacing the wavelet coefficient greater than or equal to the predetermined threshold with a difference between the wavelet coefficient and the predetermined threshold.

In a possible implementation of the embodiments of the present disclosure, the method further includes, prior to determining, from the first target HRV data, HRV data for which all RR intervals fall within the predetermined heartbeat time interval range as second target HRV data: determining an age of a person corresponding to the HRV data; and determining, based on the age of the person corresponding to the HRV data and a predetermined normal heartbeat list, a predetermined heartbeat time interval range corresponding to the age of the person. The normal heartbeat list represents correspondence between different age groups and normal heartbeat ranges.

In practice, heart rates vary among people of different age groups, and thus the corresponding RR intervals also differ. Therefore, a normal RR interval range can be determined in conjunction with the age of the person.

Specifically, normal heart rates corresponding to different age groups can refer to the predetermined normal heartbeat list: newborns (birth to 4 weeks): 100-205; infants (4 weeks to 1 year): 100-180; toddlers (1 year to 3 years): 98-140; preschoolers (3 years to 5 years): 80-120; school-age children (5 years to 12 years): 75-118; adolescents (13 years to 18 years): 60-100; adults (over 18 years): 60-100; adult athletes: 40-60.

The predetermined heartbeat time interval range is determined based on the age of the person corresponding to the HRV data and the predetermined normal heartbeat list. The RR interval=60,000/heart rate, where 60,000 represents 1 minute =60,000 milliseconds.

Whether each RR interval in the RR interval sequence falls within the predetermined heartbeat time interval range is determined. When any RR interval in the first target HRV data falls outside the predetermined heartbeat time interval range, the first target HRV data is discarded. The HRV data for which all RR intervals in the first target HRV data fall within the predetermined heartbeat time interval range is determined as the second target HRV data.

Regarding an extraction of features from the second target HRV data, the embodiments of the present disclosure provide further elaboration.

In a possible implementation, the second target HRV data is treated as a function of time. The time-domain feature of the second target HRV data is obtained through an analysis. The time-domain feature may include MeanRR (ms), MeanHR (bpm), SDNN (ms), SDANN (ms), RMSSD (ms), SDSD (ms), pNN50 (%), and pNN20 (%).

The time-domain feature of the second target HRV data includes the following features.

MeanRR: represents a mean of N RR intervals, in units of millisecond. An expression of MeanRR is:

$$MeanRR = \frac{RR_1 + RR_2 + \cdots + RR_N}{N}.$$

MeanHR: represents an average heart rate value. An expression of MeanHR is:

$$MeanHR = \frac{60000}{MeanRR}.$$

In the expression, 60000 denotes that 1 minute=60 seconds =60,000 milliseconds, and MeanRR represents the mean of N RR intervals.

SDNN: represents a standard deviation of N RR intervals, which can reflect slow changes in heart rate and is a sensitive indicator for evaluating a sympathetic nervous function. An expression of SDNN is:

$$SDNN = \sqrt{\frac{1}{N}\sum_{i=1}^{N}(RR_i - MeanRR)^2}\ .$$

In the expression, $RR_i$ represents an i-th RR interval, and MeanRR represents the mean of N RR intervals.

SDANN: represents a standard deviation of RR interval means within a predetermined time period. It is assumed that there are M sliding windows within the predetermined time period. The M sliding windows correspond to M RR interval means: MeanRR1, MeanRR2, ..., MeanRRM. An expression of SDANN is:

$$SDANN = \sqrt{\frac{1}{M}\sum_{i=1}^{M}(MeanRR_i - MeanRR_z)^2}\ .$$

In the expression, $MeanRR_z$ represents a mean of the M RR interval means. An expression of $MeanRR_z$ is:

$$MeanRR_z = \frac{MeanRR_1 + MeanRR_2 + \cdots + MeanRR_M}{M}\ .$$

RMSSD: represents a root mean square of successive adjacent RR intervals. An expression of RMSSD is:

$$RMSSD = \sqrt{\frac{1}{N}\sum_{i=1}^{N}(RR_i - RR_{i-1})^2}\ .$$

In the expression, $RR_i$ represents the i-th RR interval, and $RR_{i-1}$ represents an (i-1)-th RR interval.

SDSD: represents a standard deviation of a difference between adjacent RR intervals. An expression of SDSD is:

$$SDSD = \sqrt{\frac{1}{N}\sum_{i=1}^{N}((RR_i - RR_{i-1}) - (MeanRR - RR_{i-1}))^2}\ .$$

In the expression, $RR_i$ represents the i-th RR interval, $RR_{i-1}$ represents the (i-1)-th RR interval, N represents the quantity of RR intervals, and MeanRR represents the mean of N RR intervals.

pNN50: represents a percentage of adjacent RR intervals having a difference greater than 50 milliseconds relative to a total quantity of RR intervals. It is assumed that the quantity of RR intervals is N. An absolute value of a difference between each RR interval and a preceding RR interval is calculated, resulting in absolute values of N-1 differences. When absolute values of a differences are greater than 50 milliseconds, a value of NN50 is a. An expression of pNN50 is:

$$pNN50 = \frac{a}{N-1} \times 100\%\ .$$

pNN20: represents a percentage of adjacent RR intervals having a difference greater than 20 milliseconds relative to a total quantity of RR intervals. It is assumed that the quantity of RR intervals is N. An absolute value of a difference between each RR interval and a preceding RR interval is calculated, resulting in absolute values of N-1 differences. When absolute values of b differences are greater than 20 milliseconds, a value of NN20 is b. An expression of pNN20 is:

$$pNN20 = \frac{b}{N-1} \times 100\%$$

In a possible implementation, extracting the frequency-domain feature of the second target HRV data includes: converting the time-domain signal of the second target HRV data into the frequency-domain signal using the fast Fourier transform, and performing the feature extraction on the frequency-domain signal. The obtained frequency-domain feature may include frequency bands, including ultra-low frequency, very low frequency, low frequency, and high frequency, and frequency ranges corresponding to the frequency bands. Total Power(ms2) represents a sum of power including ultra-low frequency, very low frequency, low frequency, and high frequency. Power(ms2) represents a percentage of power of a power band of the frequency band relative to total power. Power Percent(%) represents Power Normalized, indicating a percentage of each of low frequency power and high frequency power relative to a sum of the low frequency power and the high frequency power. Peak(Hz) represents a frequency corresponding to maximum power within each frequency band. LF/HF represents a ratio of low frequency band power to high frequency band power.

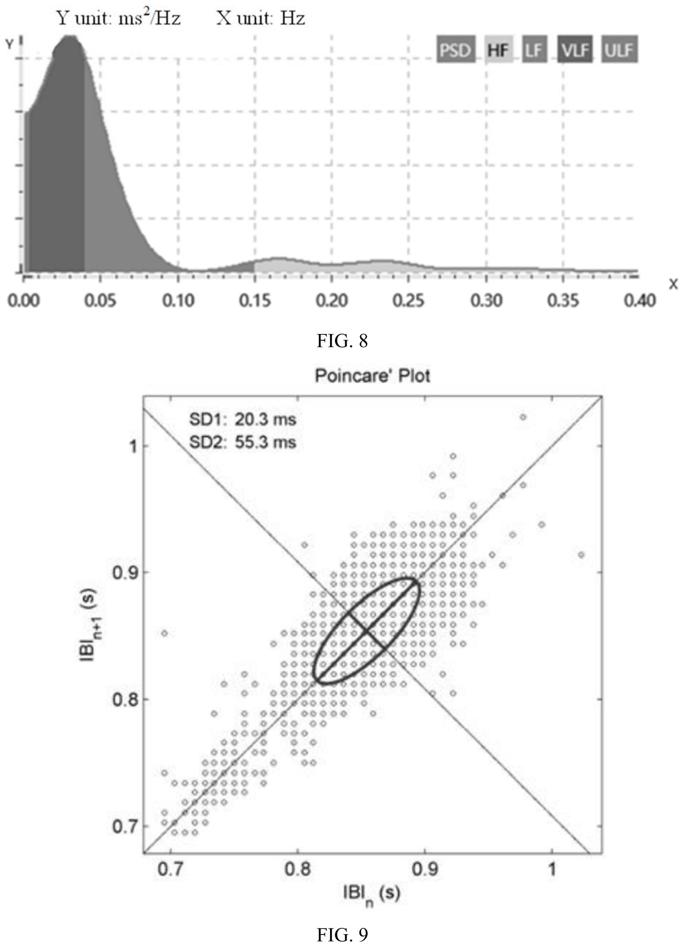

Specifically, the frequency-domain feature of the second target HRV data includes: converting the frequency-domain signal corresponding to the second target HRV data into a power spectrum from a frequency spectrum. The obtained waveform of the power spectrum is illustrated in FIG. 8. In FIG. 8, the signal is divided into four frequency bands: Ultra-Low Frequency (ULF) having a frequency ranging from 0 Hz to 0.0033 Hz; Very Low Frequency (VLF) having a frequency ranging from 0.0033 Hz to 0.04 Hz; Low Frequency (LF) having a frequency ranging from 0.04 Hz to 0.15Hz; and High Frequency (HF) having a frequency ranging from 0.15 Hz to 0.4Hz.

Power of a frequency band can be represented by an area of a graph formed by a time axis of the frequency band and a waveform, obtaining power corresponding to four frequency bands: Ultra-Low Frequency Power (PULF), Very Low Frequency Power (PVLF), Low Frequency Power (PLF), and High Frequency Power (PHF).

Total Power: represents a sum of Ultra-Low Frequency Power (PULF), Very Low Frequency Power (PVLF), Low Frequency Power (PLF), and High Frequency Power (PHF). An expression of Total Power is:

$$\text{Total Power} = P_{ULF} + P_{VLF} + P_{LF} + P_{HF}$$

Power Percent: represents a percentage of power of a frequency band relative to total power. As an example, for Ultra-Low Frequency (ULF), an expression of a percentage of power of Ultra-Low Frequency (ULF) relative to the total power is:

$$\text{Power Percent(PULF)} = \frac{PULF}{\text{Total Power}} \times 100\%$$

In the expression, Total Power represents the sum of Ultra-Low Frequency Power (PULF), Very Low Frequency Power (PVLF), Low Frequency Power (PLF), and High Frequency Power (PHF).

Power Normalized: represents the percentage of each of the low frequency power and the high frequency power relative to the sum of the low frequency power and the high frequency power. As an example, for Low Frequency (LF), an expression of a percentage of power of Low Frequency (LF) relative to the sum of the low frequency power and the high frequency power is:

$$\text{Power Normalized(PLF)} = \frac{PLF}{PLF + PHF} \times 100\%$$

In the expression, PLF represents low frequency power, and PHF represents high frequency power.

Peak: represents a frequency corresponding to the maximum power within each frequency band, which can be determined based on the power spectrum.

LF/HF: represents the ratio of the low frequency band power to the high frequency band power, i.e., PLF/PHF.

In a possible implementation of the embodiments of the present disclosure, extracting the nonlinear feature of the second target HRV data includes: drawing a Poincaré scatter plot and a difference scatter plot based on the second target HRV data; determining a standard deviation of a major axis and a standard deviation of a minor axis of an ellipse fitted to the Poincaré scatter plot; and determining a quantity of points in each of a first quadrant and a third quadrant of the difference scatter plot. The nonlinear feature includes the standard deviation of the major axis of the ellipse, the standard deviation of the minor axis of the ellipse, the quantity of points in the first quadrant, and the quantity of points in the third quadrant.

Poincaré: refers to the Poincaré scatter plot, an image drawn using variations in RR intervals. Each RR interval is influenced by a preceding RR interval. As illustrated in FIG. 9, a horizontal coordinate of the Poincaré scatter plot represents an IBI interval (RR interval), while a vertical coordinate of the Poincaré scatter plot represents a next IBI interval (RR interval). The Poincaré scatter plot reveals correlation between consecutive values in the time series, contains both linear and nonlinear changing trends in HRV, provides visual display of cardiac fluctuations, and can reveal nonlinear processes and non-periodic movements.

Nonlinear indicators extracted from the Poincaré scatter plot include the standard deviation SD1 of the major axis of the ellipse and the standard deviation SD2 of the minor axis of the ellipse.

SD1 represents short-term variability and generally reflects parasympathetic nervous activity. When SD1 decreases, the parasympathetic nervous activity decreases, and sympathetic nervous activity increases. An expression of SD1 is:

$$SD_1 = \sqrt{\frac{SDSD^2}{2}}.$$

In the expression, SDSD represents a standard deviation of a difference between adjacent RR intervals in the time-domain feature.

SD2 represents long-term variability and has a stronger association with the sympathetic nervous activity than with the parasympathetic nervous activity. When SD2 decreases, the sympathetic nervous activity increases. An expression of SD2 is:

$$SD_2 = \sqrt{2SDNN^2 - \frac{SDSD^2}{2}}.$$

In the expression, SDNN represents a standard deviation of N RR intervals in the time-domain feature, and SDSD represents the standard deviation of the differences between the adjacent RR intervals in the time-domain feature.

Scatter: also known as the difference scatter plot or a second-order difference plot, and is used to assess balance between the sympathetic nervous activity and the parasympathetic nervous activity and for a spectral analysis estimation. An advantage of Scatter is use of a stable, associated RR interval sequence. The difference scatter plot uses differences between three consecutive RR intervals to form a coordinate point for plotting. An x-axis of the difference scatter plot represents a difference between a duration $RR_i$ of a current heartbeat and a duration $RR_{i-1}$ of a previous heartbeat. A coordinate of an i-th point on the x-axis is:

$$x_i = RR_i - RR_{i-1}.$$

A y-axis of the difference scatter plot represents a difference between the duration $RR_i$ of the current heartbeat and a duration $RR_{i+1}$ of a next heartbeat. A coordinate of the i-th point on the y-axis is:

$$y_i = RR_i - RR_{i+1}.$$

The difference scatter plot represents correlation between consecutive rate values in the time series. Nonlinear indicators extracted from the difference scatter plot are the quantity A++ of points in the first quadrant and the quantity B-- of points in the third quadrant. Units of the horizontal and vertical coordinates are milliseconds.

A++: points in the first quadrant indicate an increase in two consecutive heartbeat intervals and a decrease in heart rate, representing the parasympathetic nervous activity.

B--: points in the third quadrant indicate a decrease in two consecutive heartbeat intervals and an increase in heart rate, representing the sympathetic nervous activity.

The time-domain feature, the frequency-domain feature, and the nonlinear feature that are extracted from the processed HRV data can be used for machine learning training to predict human physiological states through a trained model, improving prediction accuracy.

The above embodiments describe the HRV-data preprocessing method from a perspective of a method flow. The following embodiments describe an HRV-data preprocessing apparatus from a perspective of virtual modules or virtual units, and specific details thereof are provided in the following embodiments.

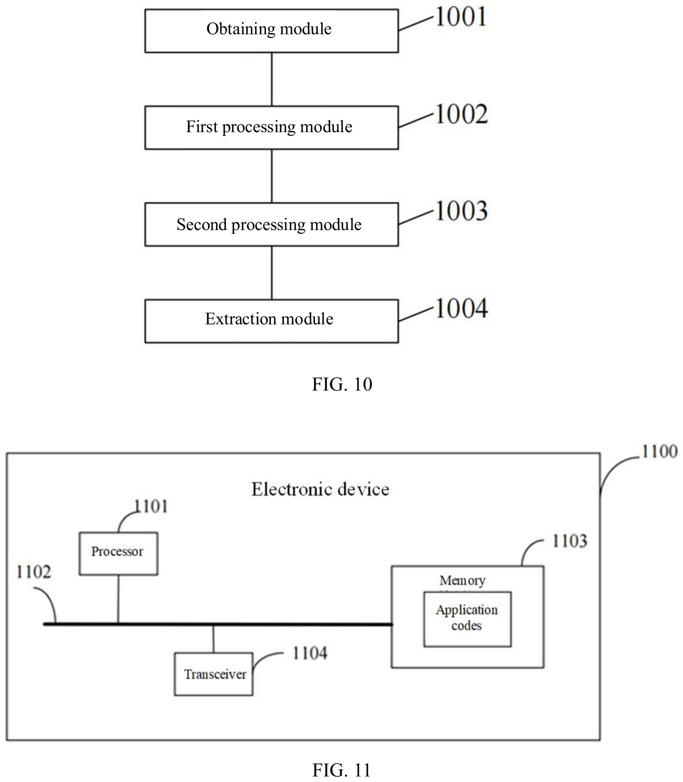

According to the embodiments of the present disclosure, an HRV-data preprocessing apparatus is provided. As illustrated in FIG. 10, the apparatus can include an obtaining module 1001, a first processing module 1002, a second processing module 1003, and an extraction module 1004. The obtaining module 1001 is configured to obtain HRV data corresponding to a sliding window. The first processing module 1002 is configured to determine, based on the HRV data, a plurality of target peaks corresponding to the HRV data, calculate a variance of the plurality of target peaks, and determine HRV data having the variance that falls within a predetermined variance range as first target HRV data. The second processing module 1003 is configured to determine an RR interval sequence of the first target HRV data based on the plurality of target peaks of the first target HRV data, determine whether each RR interval in the RR interval sequence falls within a predetermined heartbeat time interval range, and determine, from the first target HRV data, HRV data for which all RR intervals fall within the predetermined heartbeat time interval range as second target HRV data. The extraction module 1004 is configured to extract a time-domain feature, a frequency-domain feature, and a nonlinear feature of the second target HRV data.

In an optional example, the present disclosure can be further configured as follows. The first processing module 1002 is specifically configured to, in response to determining, based on the HRV data, the plurality of target peaks corresponding to the HRV data: determine a plurality of initial peaks in the HRV data based on a minimum cardiac cycle point count, in which the minimum cardiac cycle point count represents a quantity of data points included in one heartbeat in the HRV data; determine a first upper envelope and a first lower envelope of the HRV data; determine a voltage threshold of the HRV data based on a percentage threshold, the first upper envelope, and the first lower envelope; and select, from the plurality of initial peaks, peaks exceeding the voltage threshold to obtain the plurality of target peaks of the HRV data.

In an optional example, the present disclosure can be further configured as follows. The obtaining module 1001 is specifically configured to, in response to obtaining the HRV data corresponding to the sliding window: obtain first initial HRV data corresponding to the sliding window, and determine a second upper envelope and a second lower envelope of the first initial HRV data; determine a maximum amplitude difference between the second upper envelope and the second lower envelope; determine an amplitude adjustment ratio of each data point in the first initial HRV data based on a second upper envelope value and a second lower envelope value that correspond to each data point in the first initial HRV data, and the maximum amplitude difference; and perform an amplitude adjustment on each data point in the first initial HRV data based on the amplitude adjustment ratio corresponding to the data point to obtain the HRV data.

In an optional example, the present disclosure can be further configured as follows. The obtaining module 1001 is specifically configured to, in response to performing the amplitude adjustment on each data point in the first initial HRV data based on the amplitude adjustment ratio corresponding to the data point to obtain the HRV data: perform the amplitude adjustment on each data point in the first initial HRV data based on the amplitude adjustment ratio corresponding to the data point to obtain amplitude-adjusted first HRV data; perform an anomaly detection on the amplitude-adjusted first HRV data to determine an anomalous point; and correct a value of the anomalous point based on a mean or a median corresponding to a plurality of data points preceding or following the anomalous point to obtain the HRV data.

In an optional example, the present disclosure can be further configured as follows. The obtaining module 1001 is specifically configured to, in response to performing the anomaly detection on the amplitude-adjusted first HRV data to determine the anomalous point: determine, in the amplitude-adjusted first HRV data, a first difference between a target data point and a preceding data point and a second difference between the target data point and a following data point, in which the target data point is any data point in the first HRV data; calculate a first ratio of the first difference to the target data point and a second ratio of the second difference to the target data point; and when each of the first ratio and the second ratio for the target data point is greater than a predetermined ratio threshold, determine the target data point as the anomalous point.

In an optional example, the present disclosure can be further configured as follows. The obtaining module 1001 is specifically configured to, in response to performing the anomaly detection on the amplitude-adjusted first HRV data to determine the anomalous point: determine a median of the amplitude-adjusted first HRV data, and determine a median deviation of a target data point in the amplitude-adjusted first HRV data based on the median; and when the median deviation of the target data point is greater than a predetermined deviation threshold, determine the target data point as the anomalous point.

In an optional example, the present disclosure can be further configured as follows. The obtaining module 1001 is specifically configured to, in response to obtaining the first initial HRV data corresponding to the sliding window: obtain second initial HRV data corresponding to the sliding window; perform a fast Fourier transform on a time-domain signal corresponding to the second initial HRV data to obtain a frequency-domain signal corresponding to the second initial HRV data; determine whether the frequency-domain signal corresponding to the second initial HRV data is a valid frequency-domain signal based on a predetermined frequency-domain range; and perform segmented filtering on the valid frequency-domain signal when the frequency-domain signal corresponding to the second initial HRV data is the valid frequency-domain signal, integrate the segmented-filtered valid frequency-domain signal, and perform an inverse fast Fourier transform on the integrated signal to obtain filtered first initial HRV data.

In an optional example, the present disclosure can be further configured as follows. The extraction module 1004 is specifically configured to, in response to extracting the nonlinear feature of the second target HRV data: draw a Poincaré scatter plot and a difference scatter plot based on the second target HRV data; determine a standard deviation of a major axis and a standard deviation of a minor axis of an ellipse fitted to the Poincaré scatter plot; and determine a quantity of points in each of a first quadrant and a third quadrant of the difference scatter plot. The nonlinear feature includes the standard deviation of the major axis of the ellipse, the standard deviation of the minor axis of the ellipse, the quantity of points in the first quadrant, and the quantity of points in the third quadrant.

In an optional example, the present disclosure can be further configured as follows. The plurality of target peaks are peaks in the HRV data that exceed a voltage threshold. The first processing module 1002 is configured to determine a maximum amplitude difference between a first upper envelope and a first lower envelope of the HRV data, determine a product of the maximum amplitude difference and a predetermined percentage threshold, and determine a sum of the product and an amplitude of the first lower envelope corresponding to the maximum amplitude difference as the voltage threshold.

Those skilled in the art can clearly understand that, for convenience and brevity of description, the specific working process of the HRV-data preprocessing apparatus described above can refer to the corresponding process in the above method embodiments, and thus details thereof will be omitted here.

An electronic device is provided according to an embodiment of the present disclosure. As illustrated in FIG. 11, an electronic device 1100 illustrated in FIG. 11 includes a processor 1101 and a memory 1103. The processor 1101 and the memory 1103 are connected, such as via a bus 1102. Alternatively, the electronic device 1100 may further include a transceiver 1104. It should be noted that, in practical applications, the electronic device 1100 is not limited to including one transceiver 1104. A structure of the electronic device 1100 does not constitute a limitation to the embodiments of the present disclosure.

The processor 1101 may be a Central Processing Unit (CPU), a general purpose processor, a Digital Signal Processor (DSP), an Application Specific Integrated Circuit (ASIC), a Field Programmable Gate Array (FPGA) or other programmable logic devices, a transistor logic device, a hardware component, or any combination thereof. The processor 1101 can implement or perform various illustrative logical blocks, modules, and circuits described in connection with the present disclosure. The processor 1101 may also be a combination that implements computing functions, such as a combination including one or more microprocessors, a combination including a DSP and a microprocessor, and the like.

The bus 1102 may include a pathway for transmitting information among the above-mentioned components. The bus 1102 may be a Peripheral Component Interconnect (PCI) bus, an Extended Industry Standard Architecture (EISA) bus, or the like. The bus 1102 may be categorized into an address bus, a data bus, a control bus, etc. For ease of illustration, only one thick line is used in FIG. 11 to represent the bus, but this does not imply that there is only one bus or one type of bus.

The memory 1103 may be a Read Only Memory (ROM) or other types of static storage devices capable of storing static information and instructions, a Random Access Memory (RAM) or other types of dynamic storage devices capable of storing information and instructions, an Electrically Erasable Programmable Read Only Memory (EEPROM), a Compact Disc Read Only Memory (CD-ROM) or other optical disc storage, optical disc storage (including a compact disc, a laser disc, an optical disc, a digital versatile disc, a Blu-ray disc, etc.), a magnetic disk storage medium or other magnetic storage devices, or any other medium capable of carrying or storing desired program codes in the form of instructions or data structures and accessible by a computer, but is not limited thereto.

The memory 1103 is configured to store application codes for implementing the solutions of the present disclosure, and is controlled and executed by the processor 1101. The processor 1101 is configured to execute application codes stored in the memory 1103 to implement the content illustrated in the above embodiments of the HRV-data preprocessing method.

The electronic device includes, but is not limited to: a mobile phone, a notebook computer, a digital broadcast receiver, a Personal Digital Assistant (PDA), a tablet computer, a Portable Multimedia Player (PMP), a mobile terminal such as a vehicle-mounted terminal (e.g., a vehicle-mounted navigation terminal), and a fixed terminal such as a digital TV and a desktop computer. It may also be a server. The electronic device illustrated in FIG. 11 is merely an example and should not impose any limitation on the functionality or scope of use of the embodiments of the present disclosure.

According to the embodiments of the present disclosure, a computer-readable storage medium is provided. The computer-readable storage medium stores computer programs or computer instructions. The computer programs or computer instructions, when executed by a processor, implement corresponding contents in the above method embodiments. The computer-readable storage medium may be a tangible storage medium, such as a Random Access Memory (RAM), a memory, a Read Only Memory (ROM), an electrically programmable ROM, an electrically erasable programmable ROM, a register, a floppy disk, a hard disk, a removable storage disk, a CD-ROM, or any other form of storage medium well known in the art.

The present disclosure further provides an apparatus. The apparatus includes a processor configured to invoke and execute a computer program from a memory, enabling a device equipped with the apparatus to perform the corresponding content as implemented in the above method embodiments.

The present disclosure further provides a computer program product, including a computer program or computer instructions. The computer program or the computer instructions, when executed by a processor, implement the corresponding content in the above method embodiments.

It should be understood that, although steps in each of the flowcharts in the figures are illustrated in a sequence indicated by an arrow, these steps are not necessarily executed in the sequence indicated by the arrow. Unless explicitly stated herein, execution of these steps is not strictly limited to the sequence, and these steps may be performed in other sequences. In addition, at least a part of the steps in each of the flowcharts in the figures may include several sub-steps or several stages. These sub-steps or stages are not necessarily executed and completed at the same time, but may be executed at different times. These sub-steps or stages are also not necessarily executed sequentially one by one, but may be executed in turn or alternately with other steps, or sub-steps of other steps, or at least a part of the stages.

Only some embodiments of the present disclosure are described above. It should be noted that, for those skilled in the art, several improvements and modifications can be made without departing from the principles of the present disclosure. These improvements and modifications shall fall within the protection scope of the present disclosure.

**Claims**

1. An HRV-data preprocessing method, comprising:

   obtaining HRV data corresponding to a sliding window;
   determining, based on the HRV data, a plurality of target peaks corresponding to the HRV data, calculating a variance of the plurality of target peaks, and determining HRV data having the variance that falls within a predetermined variance range as first target HRV data;
   determining an RR interval sequence of the first target HRV data based on the plurality of target peaks of the first target HRV data, determining whether each RR interval in the RR interval sequence falls within a predetermined heartbeat time interval range, and determining, from the first target HRV data, HRV data for which all RR intervals fall within the predetermined heartbeat time interval range as second target HRV data; and
   extracting a time-domain feature, a frequency-domain feature, and a nonlinear feature of the second target HRV data.

2. The method according to claim 1, wherein said determining, based on the HRV data, the plurality of target peaks corresponding to the HRV data comprises:

   determining a plurality of initial peaks in the HRV data based on a minimum cardiac cycle point count, wherein the minimum cardiac cycle point count represents a quantity of data points comprised in one heartbeat in the HRV data;
   determining a first upper envelope and a first lower envelope of the HRV data;
   determining a voltage threshold of the HRV data based on a percentage threshold, the first upper envelope, and the first lower envelope; and
   selecting, from the plurality of initial peaks, peaks exceeding the voltage threshold to obtain the plurality of target peaks of the HRV data.

3. The method according to claim 1, wherein said obtaining the HRV data corresponding to the sliding window comprises:

   obtaining first initial HRV data corresponding to the sliding window, and determining a second upper envelope and a second lower envelope of the first initial HRV data;
   determining a maximum amplitude difference between the second upper envelope and the second lower envelope;
   determining an amplitude adjustment ratio of each data point in the first initial HRV data based on a second upper envelope value and a second lower envelope value that correspond to each data point in the first initial HRV data,

and the maximum amplitude difference; and
performing an amplitude adjustment on each data point in the first initial HRV data based on the amplitude adjustment ratio corresponding to the data point to obtain the HRV data.

4. The method according to claim 3, wherein said performing the amplitude adjustment on each data point in the first initial HRV data based on the amplitude adjustment ratio corresponding to the data point to obtain the HRV data comprises:

performing the amplitude adjustment on each data point in the first initial HRV data based on the amplitude adjustment ratio corresponding to the data point to obtain amplitude-adjusted first HRV data;
performing an anomaly detection on the amplitude-adjusted first HRV data to determine an anomalous point; and
correcting a value of the anomalous point based on a mean or a median corresponding to a plurality of data points preceding or following the anomalous point to obtain the HRV data.

5. The method according to claim 4, wherein said performing the anomaly detection on the amplitude-adjusted first HRV data to determine the anomalous point comprises:

determining, in the amplitude-adjusted first HRV data, a first difference between a target data point and a preceding data point and a second difference between the target data point and a following data point, wherein the target data point is any data point in the first HRV data;
calculating a first ratio of the first difference to the target data point and a second ratio of the second difference to the target data point; and
when each of the first ratio and the second ratio for the target data point is greater than a predetermined ratio threshold, determining the target data point as the anomalous point.

6. The method according to claim 4, wherein said performing the anomaly detection on the amplitude-adjusted first HRV data to determine the anomalous point comprises:

determining a median of the amplitude-adjusted first HRV data, and determining a median deviation of a target data point in the amplitude-adjusted first HRV data based on the median; and
when the median deviation of the target data point is greater than a predetermined deviation threshold, determining the target data point as the anomalous point.

7. The method according to claim 3, wherein said obtaining the first initial HRV data corresponding to the sliding window comprises:

obtaining second initial HRV data corresponding to the sliding window;
performing a fast Fourier transform on a time-domain signal corresponding to the second initial HRV data to obtain a frequency-domain signal corresponding to the second initial HRV data;
determining whether the frequency-domain signal corresponding to the second initial HRV data is a valid frequency-domain signal based on a predetermined frequency-domain range; and
performing segmented filtering on the valid frequency-domain signal when the frequency-domain signal corresponding to the second initial HRV data is the valid frequency-domain signal, integrating the segmented-filtered valid frequency-domain signal, and performing an inverse fast Fourier transform on the integrated signal to obtain filtered first initial HRV data.

8. The method according to claim 1, wherein:

the plurality of target peaks are peaks in the HRV data that exceed a voltage threshold; and
the voltage threshold is determined by:
determining a maximum amplitude difference between a first upper envelope and a first lower envelope of the HRV data, determining a product of the maximum amplitude difference and a predetermined percentage threshold, and determining a sum of the product and an amplitude of the first lower envelope corresponding to the maximum amplitude difference as the voltage threshold.

9. The method according to any one of claims 1 to 8, wherein said extracting the nonlinear feature of the second target HRV data comprises:

drawing a Poincaré scatter plot and a difference scatter plot based on the second target HRV data;
determining a standard deviation of a major axis and a standard deviation of a minor axis of an ellipse fitted to the Poincaré scatter plot; and
determining a quantity of points in each of a first quadrant and a third quadrant of the difference scatter plot, wherein the nonlinear feature comprises the standard deviation of the major axis of the ellipse, the standard deviation of the minor axis of the ellipse, the quantity of points in the first quadrant, and the quantity of points in the third quadrant.

10. An HRV-data preprocessing apparatus, comprising:

an obtaining module configured to obtain HRV data corresponding to a sliding window;
a first processing module configured to determine, based on the HRV data, a plurality of target peaks corresponding to the HRV data, calculate a variance of the plurality of target peaks, and determine HRV data having the variance that falls within a predetermined variance range as first target HRV data;
a second processing module configured to determine an RR interval sequence of the first target HRV data based on the plurality of target peaks of the first target HRV data, determine whether each RR interval in the RR interval sequence falls within a predetermined heartbeat time interval range, and determine, from the first target HRV data, HRV data for which all RR intervals fall within the predetermined heartbeat time interval range as second target HRV data; and
an extraction module configured to extract a time-domain feature, a frequency-domain feature, and a nonlinear feature of the second target HRV data.

11. An electronic device, comprising:

at least one processor;
a memory; and
at least one application stored in the memory and configured to be executed by the at least one processor to implement the HRV-data preprocessing method according to any one of claims 1 to 9.

12. An apparatus, comprising:
a processor configured to invoke and execute a computer program from a memory, enabling a device equipped with the apparatus to perform the HRV-data preprocessing method according to any one of claims 1 to 9.

13. A computer-readable storage medium, having a computer program or computer instructions stored thereon, wherein the computer program or the computer instructions, when executed by a processor, implement the HRV-data preprocessing method according to any one of claims 1 to 9.

14. A computer program product, comprising a computer program or computer instructions, wherein the computer program or the computer instructions, when executed by a processor, implement the HRV-data preprocessing method according to any one of claims 1 to 9.

HRV data corresponding to a sliding window is obtained — S101

A plurality of target peaks corresponding to the HRV data are determined based on the HRV data, a variance of the plurality of target peaks is calculated, and HRV data having the variance that falls within a predetermined variance range is determined as first target HRV data — S102

An RR interval sequence of the first target HRV data is determined based on the plurality of target peaks of the first target HRV data, whether each RR interval in the RR interval sequence falls within a predetermined heartbeat time interval range is determined, and HRV data for which all RR intervals fall within the predetermined heartbeat time interval range is determined from the first target HRV data as second target HRV data — S103

A time-domain feature, a frequency-domain feature, and a nonlinear feature of the second target HRV data are extracted — S104

FIG. 1

FIG. 2

FIG. 3

FIG. 4

FIG. 5

FIG. 6

FIG. 7

Y unit: ms²/Hz     X unit: Hz

FIG. 8

Poincare' Plot

SD1: 20.3 ms
SD2: 55.3 ms

FIG. 9

Obtaining module ⟋ 1001

First processing module ⟋ 1002

Second processing module ⟋ 1003

Extraction module ⟋ 1004

FIG. 10

Electronic device ⟋ 1100

Processor ⟋ 1101

1102

Memory ⟋ 1103

Application codes

Transceiver ⟋ 1104

FIG. 11

## INTERNATIONAL SEARCH REPORT

| International application No. |
|---|
| **PCT/CN2024/095337** |

### A. CLASSIFICATION OF SUBJECT MATTER

G06F18/10(2023.01)i

According to International Patent Classification (IPC) or to both national classification and IPC

### B. FIELDS SEARCHED

Minimum documentation searched (classification system followed by classification symbols)

IPC: G06F

Documentation searched other than minimum documentation to the extent that such documents are included in the fields searched

Electronic data base consulted during the international search (name of data base and, where practicable, search terms used)

VEN, CNABS, CNTXT, WOTXT, EPTXT, USTXT, CNKI, IEEE: 数据, 预处理, 心率, 峰值, 准确度, 时域, 频域, data, process, heart, rate, HRV

### C. DOCUMENTS CONSIDERED TO BE RELEVANT

| Category* | Citation of document, with indication, where appropriate, of the relevant passages | Relevant to claim No. |
|---|---|---|
| PX | CN 117271977 A (KINGFAR INTERNATIONAL INC.) 22 December 2023 (2023-12-22) description, paragraphs [0005]-[0133] | 1-14 |
| X | CN 111067503 A (SHENZHEN GLORY INFORMATION TECHNOLOGY CO., LTD. et al.) 28 April 2020 (2020-04-28) description, paragraphs [0005]-[0172] | 1-14 |
| A | CN 105574348 A (NORTHWESTERN POLYTECHNICAL UNIVERSITY) 11 May 2016 (2016-05-11) entire document | 1-14 |
| A | JP 2012065713 A (GIFU UNIVERSITY) 05 April 2012 (2012-04-05) entire document | 1-14 |

☐ Further documents are listed in the continuation of Box C.    ☑ See patent family annex.

| | |
|---|---|
| *   Special categories of cited documents:<br>"A"   document defining the general state of the art which is not considered to be of particular relevance<br>"D"   document cited by the applicant in the international application<br>"E"   earlier application or patent but published on or after the international filing date<br>"L"   document which may throw doubts on priority claim(s) or which is cited to establish the publication date of another citation or other special reason (as specified)<br>"O"   document referring to an oral disclosure, use, exhibition or other means<br>"P"   document published prior to the international filing date but later than the priority date claimed | "T"   later document published after the international filing date or priority date and not in conflict with the application but cited to understand the principle or theory underlying the invention<br>"X"   document of particular relevance; the claimed invention cannot be considered novel or cannot be considered to involve an inventive step when the document is taken alone<br>"Y"   document of particular relevance; the claimed invention cannot be considered to involve an inventive step when the document is combined with one or more other such documents, such combination being obvious to a person skilled in the art<br>"&"   document member of the same patent family |

| Date of the actual completion of the international search | Date of mailing of the international search report |
|---|---|
| **21 August 2024** | **27 August 2024** |

| Name and mailing address of the ISA/CN | Authorized officer |
|---|---|
| **China National Intellectual Property Administration (ISA/CN)**<br>**China No. 6, Xitucheng Road, Jimenqiao, Haidian District, Beijing 100088** | |
| | Telephone No. |

Form PCT/ISA/210 (second sheet) (July 2022)

## INTERNATIONAL SEARCH REPORT
### Information on patent family members

International application No.

**PCT/CN2024/095337**

| Patent document cited in search report | | | Publication date (day/month/year) | Patent family member(s) | Publication date (day/month/year) |
|---|---|---|---|---|---|
| CN | 117271977 | A | 22 December 2023 | None | |
| CN | 111067503 | A | 28 April 2020 | None | |
| CN | 105574348 | A | 11 May 2016 | None | |
| JP | 2012065713 | A | 05 April 2012 | None | |

Form PCT/ISA/210 (patent family annex) (July 2022)

**REFERENCES CITED IN THE DESCRIPTION**

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- CN 202311265053